# EUROPEAN PATENT APPLICATION

(11) **EP 1 825 850 A1**
(43) Date of publication of application: **29.08.2007**
(21) Application number: 06003757.9
(22) Date of filing: 24.02.2006
(51) Int. Cl.: A61K 31/05, A61P 3/00, A61P 9/00, A61P 29/00, A61P 37/00, A61P 39/00

(54) **Use of resveratrol and derivatives thereof for promoting the wellness state in mammals**

(71) Applicant: DSM IP Assets B.V., 6411 TE Heerlen (NL)
(72) Inventor: Raederstorff, Daniel, 68720 Flaxlanden (FR); Schehlmann, Volker, 79650 Schopfheim (DE); Wang-Schmidt, Ying, 8302 Kloten (CH); Weber, Gilbert, 4312 Magden (CH)
(74) Representative: Grossner, Lutz

(57) **Abstract**

The invention relates to the use of resveratrol, a derivative, metabolite or analogue thereof for maintaining and/or promoting the wellness state of a mammal and for changing gene expression profiles in adult mammals towards conformity with expression profiles of such genes found in younger adult mammals ; and to the use of resveratrol, a derivative, metabolite or analogue thereof in the manufacture of nutraceutical compositions for such purposes.

## Description

The present invention refers to a method of promoting the wellness state of an organism by inducing physiological changes that will give the organism a younger molecular phenotype. The expression "organism" is to be understood to refer to a mammal which comprises human beings and animals as well. Mammals in the context of the present invention include humans, other primates, carnivore, artiodactyla, rodents, perissodactyla, lagomorpha etc. Preferred "mammals" are humans and pets such as cats, dogs and horses.

Promotion of the wellness state, in the context of the present invention, denotes the promotion and enhancement of the global health state of an organism including both physical health and mental health.

Physical health is a state of general good body health in which all functions of an organism are in optimal working conditions, being free of activity limitations and chronic disorders. The expression "disorder" is used herein to also encompass diseases. Thus, mobility, physical performance, tolerance to thermal stress is maintained. It is being preserved from physical disorders, being not damaged and free of pain.

Mental health is a state of optimal performance of mental function, resulting in learning and productive activities, social connectivity to other individuals, and ability to adapt to change and to cope with adversity and stress. Mental health is also the state of being free of mental disorders such as depression, anxiety, cognitive problems, and alterations in thinking, mood or behavior.

Wellness denotes the global health state (physical and mental health) of an organism., especially when maintainted by proper diet, exercise and habits (see The American Heritage® Stedman's Medical Dictionary, publ. Houghton Mifflin Co, 2002). However, such pre-conditions for maintaining the state of wellness are not fulfilled for too many in today's population. The wellness state is a continuum for both physical and mental health from being completely healthy to completely ill and wellness can be promoted along this continuum. Wellness includes diseases prevention and health promotion. The state of wellness can be determined, inter alia, by evaluating measurable parameters such as body functions, e.g., blood pressure, heart rate, body fat composition, pulmonary function, liver function, brain function and levels of physiologically vital components in body fluids etc. as disclosed in US patent 5,692,501.

Key factors for the global health/ wellness state of an organism are the capacity of an organism to maintain homeostasis, repair and regeneration capacity.

Homeostasis is the inherent tendency in an organism toward maintenance of physiological and psychological stability. Homeostatic systems in mammals are for example: the regulation of body temperature, the regulation of the amounts of water and minerals in the body, the removal of metabolic waste, the regulation of blood glucose and lipid levels. At the cellular level factors like for example temperature, salinity, acidity, the concentrations of nutrients such as glucose, lipids, various ions (calcium, sodium, potassium..), oxygen, and wastes, such as carbon dioxide and urea must also be maintained within tolerable limits. In multi-cellular organisms those factors also have to be maintained at desirable levels in body fluids such as blood plasma, tissue fluid and intracellular fluid to allow the organism to function more effectively. Thus especially complex systems like the human body must have efficient homeostatic systems to be able to adapt to modifications of the environments and to maintain stability and a state of wellness in the body. The above mentioned factors when outside of safe windows have a negative impact on metabolism, which are all the biochemical processes of an organism that keep bodies healthy and alive. This dysregulation of cellular homeostasis will then induce a gradual decline of organ functions. Therefore, there are built-in physiological mechanisms (homeostatic systems) to maintain the factors at desirable levels. Homeostasis is not a static state but a state of equilibrium. At the cellular level homeostasis must be maintained in the presence of a constant metabolic flux of molecules. For example the cellular components like protein, membranes, sugars and nucleic acids are constantly recycled while the integrity of the organism as a whole is preserved by homeostatic systems. In an organism challenge by multiple internal and external stimuli the homeostatic systems must be robust and stable to preserve the proper functioning of its component cells, organs, organ systems and whole body. Thus homeostasis systems are essential control mechanisms that maintain the internal environment of an organism within tolerable limits to maintain health and optimal function. This occurs by regulating the metabolism of an organism through numerous metabolic pathways which are series of chemical reactions occurring in cells or whole organisms. The metabolism of an organism can be divided in two main processes the biosynthesis of molecules (anabolism) in which cells use energy to build complex molecules, cellular structure and perform the organism functions and the process for breaking down molecules to produce energy (catabolism). The whole organism must also maintain homeostasis between catabolism and anabolism.
If the body loses its normal homeostasis, adverse symptoms appear with a process trying to bring the body back into balance if this is not successful over time disorders will develop. Therefore, chronic imbalances of the normal metabolic pathways are the causes for the development of numerous disorders.

The regulation of metabolism and metabolic pathways to maintain homeostasis are central to the molecular events resulting in health and optimal function or disorders. The cellular metabolism is linked to the cellular health and the wellness of an organism is correlated to the cellular function in that organism. The cellular metabolism is maintained in a state of dynamic equilibrium (homeostasis) by interrelated complex metabolic pathways. The cellular responses to stimuli are often a result of coordinated activity of group of genes which tend to maintain homeostasis. Thus, the cells respond to internal and external stimuli by change in the gene expression profiles.

The cellular metabolism can directly and profoundly influence gene expression which in turn will affect cellular metabolism by a feedback mechanism and such the gene expression profile modulate the function of an organism by regulating the metabolic pathways in the cells. Thus a change in gene expression will have an impact on cellular metabolism and vise versa. The gene expression profiles can be used as a global marker of the status and response of cells, organs and bodies, which result from changes in the status of proteins and other metabolites. The gene expression profile can also be used to identify specific metabolic processes and cellular functions that are changed between different individuals. The global gene expression profiling will define the genome wide response occurring during cellular metabolism and the changes in gene expression reflect changes in cellular function.

The global gene expression profiles give a overview of the global cellular activities and functions reflecting the physiological state and wellness state of an organism.

The Microarray technology allows a genome wide gene expression analysis to determine the global gene expression in tissues and assess the gene regulation in an organism. The microarray technology can be used to analyze the expression pattern and the expression levels of thousands of genes simultaneously. Thus, the transciptional status of most of the genes of the genome is determined. The technique also allows a direct quantitative comparison of the expression level of specific genes in the same tissues from organisms under different physiological conditions and will give information on the physiological state of that organism at the molecular level. The gene expression changes provide the molecular phenotype of a tissue which can be used to discriminate between normal and pathological states as well as to determine the effect of certain intervention on such physiological states. When applied to nutritional intervention, the microarray technology is a highly effective way to understand the function of dietary nutrients and the global response of the body to those nutrients.

The cellular responses to external stimuli or intracellular fluxes of molecules are often transient but can have profound impact on cellular function. The modulation of gene expression is an early and rapid response of the cell to those challenges and change in the cellular process. Thus, the coordinated regulation of gene expression is essential for a cell or an organism to respond successfully to external or internal stimuli and adapt to changing intracellular environments and maintain homeostasis.

A disorder is usually diagnosed by measuring various biomarkers. However, a disorder that results from a long term imbalances in metabolism may not have a measurable biomarker of damage before the disorders is well established. Thus often when the physician makes a diagnosis the diseases is already present. Moreover, it is often not possible to reverse chronic disorders by simply restoring normal balance of specific aspects of metabolism. The change in gene expression is an early event in reaction to a challenge or change in the cell process such allowing the detection and correction of any undesired structural changes at the molecular level and at a very early stage before any damage as occurred to maintain an organism in an optimal physiological state or wellness state.

The key factors known to make an undamaged young adult organism more fit and in a better state of wellness than an average old organism are a better capacity to maintain homeostasis, a more efficient repair system and a more rapid regeneration capacity. For example young organisms have a higher capacity than old organisms to repair and regenerate tissues such as liver, muscle, bone, arterial wall. Young adult organisms are more resistant to stress by being able to maintain cellular homeostasis up to a higher stress challenge than old organisms. Young organisms have sensitive homeostatic systems which, in comparison to older organisms, can respond more rapidly to an imbalance in cellular metabolism and repair the damage faster such also decreasing the recovery period after a challenge. Young adult organisms also have better homeostatic systems by having a wider dynamic range of condition within which the organism can properly function such for example they have a better thermo-regulation and are more resistant to a heat choc (thermal stress) than corresponding older organisms. Organisms in a young state also have a higher capacity to maintain the body in a state of homeostasis by removing more rapidly damaged cells and molecules and having a faster repair and regenerative capacity such maintaining the body in a better wellness state.

The link between the global gene expression profile and the physiological function of an organ has recently been shown in humans by Rodwell et al. (PLOS Biology (2004), 2 (12) 2191-2201). In this study the gene expression profiles correlated well with the morphological and physiological state of the kidney in humans. Moreover, they showed that older humans with a gene expression profile normally associated with younger people tended to have a kidney in better condition for their age with a morphological appearance and a physiological state more similar to the one from younger people. In the contrary a human with gene expression profiles normally associated with people much older had a kidney in poorer condition for their age with a morphological appearance and a physiological state more similar to the one from much older people.

The results indicate that the tissue gene expression profiles are able to predict if humans have kidneys exhibiting unusual state of wellness or abnormal degeneration for their respective chronological age. Finally, in two different types of human kidney tissues from old and young human samples the same gene varied suggesting that the same molecular difference between old and young cells would occur in all organs and that there is a common way for all tissues to get old.

Genes involved in the maintenance of normal physiological values of blood cholesterol (lipids), blood triglyceride lipids, blood Low Density Lipoprotein (LDL) and High Density Lipoprotein (HDL) and the LDL to HDL ratio, blood glucose, liver function, heart rate, protein, vitamin and mineral metabolism, immune system natural killer cell (NK) activity, immune system vitality and proportion of NK cells , immune cells (NK, B and T-cell counts) and immune T-cell helper/suppressor ratio ; and genes involved in apoptosis, cell adhesion, cell growth and maintenance, cytoskeletal organization, embryonal development, electron transport, endo-exophagocytosis, inflammation/immune response, metabolism of carbohydrates, fatty acids, lipids, nucleic acids, TCA cycle, protein folding, protein synthesis, protein ubiquination, proteolysis, response to stress, signal transduction, transcription, or transport may be regarded as contributing especially to maintaining and promoting the state of wellness.

In terms of gene expression products, genes involved in the expression of IgF1r, Bcl2 antagonist, cyclooxygenase, epsecially genes involved in protein synthesis, turnover and modification, such as eIF4A, 4E, 4gamma1,elF3subunit10, eukaryotic translation elongation factor 2, mitochondrial ribosomal protein L43, L27, ARF binding protein3, f-box only protein 9, DnaJ (Hsp40 homolog, Hsp1beta etc. are vital to maintenance and promotion of wellness.

Thus, the gene expression profile may be used as an indicator of the wellness state of an organism. The higher capacity to keep the metabolic process in balance and to maintain homeostasis in young adult as compared to a corresponding older subject may be reflected in the gene expression profiles. Thus, the average gene expression profile of an young adult organism may be used as reference for an optimal physiological state of wellness. Moreover, the state of wellness of an organism could be promoted by creating a younger environment for the cell by changing the cellular signaling to a younger profile and such restoring the capacity to maintain homeostasis and the regenerative efficiency. The genome wide gene analysis of the gene expression profile allows a global approach to assess the wellness state of a cell, a tissue or an organism. The comparison of the gene expression profile of an organism to that of an healthy younger adult organism may be used as a measure of the global wellness sate of the organism. A younger gene expression profile reflects a younger metabolic and signaling profile of cell or organism, which will be more resistant to internal or external stimuli and thus, maintain the organism in a better wellness state.

It has been found in accordance with the invention that promotion of the wellness state of a mammal can be achieved by administering to said mammal an effective amount of resveratrol, a derivative, metabolite or analogue thereof.

The term "resveratrol, a derivative, metabolite or analogue thereof" as used herein comprises compounds encompassed by the general formula I 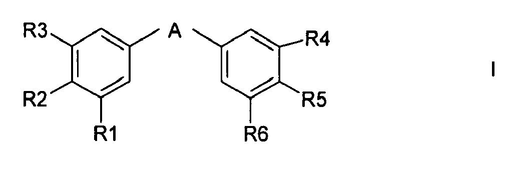 wherein A denotes a carbon-carbon double bond which may be trans or cis, and R1, R2, R3, R4, R5 and R6, independently denote hydrogen, hydroxy or etherified or esterified hydroxy groups.

While the carbon-carbon double bond denoted by the symbol A may be trans or cis, formula I above is understood to also include cis/trans mixtures. However, compounds of formula I wherein A is a trans carbon-carbon bond are preferred.

Etherified or esterified hydroxy groups may be derived from unsubstituted or substituted, straight or branched chain alkyl groups having 1 to 26 carbon atoms or from unsubstituted or substituted, straight or branched chain aliphatic, araliphatic or aromatic carboxylic acids having 1 to 26 carbon atoms. Etherified hydroxy groups may further be glycoside groups and esterified hydroxy groups may further be glucuronide or sulfate groups. Examples of compounds of formula I are resveratrol (R1, R3 and R5 = hydrogen, R2, R4 and R6 = hydroxy); piceatannol (R3 and R5 = hydrogen, R1, R2, R4 and R6 = hydroxy), and rhapontigenin (R5 = hydrogen, R1, R3, R4 and R6 = hydroxy, and R2 = methoxy).

For the purposes of the invention, resveratrol may be of synthetic or of natural origin. In one embodiment of the invention, resveratrol, particularly (trans)-resveratrol of synthetic origin is used for the purposes of the invention. In another embodiment of the invention, resveratrol of natural origin is used, e.g., as a resveratrol-containing extract from natural resveratrol sources such as grape seed extract or giant knotweed extract. Furthermore, resveratrol may be used for the purposes of the invention in combination with one or more active ingredients conventionally used in nutritional supplemental formulations, such as mineral salts; vitamins, e.g., vitamin E and C; carotenoids such as β-carotene, lycopene, lutein or zeaxanthin; green tea catechin such as epigallocatechin; olive phenolics such as hydroxytyrosol and oleuropein; Coenzyme Q10; and genistein. However, resveratrol may be used for the purposes of the invention as the sole active ingredient.

In accordance with the present invention it has been found that the gene expression profile of a mammal whose diet is supplemented with resveratrol, a derivative, metabolite or analogue thereof is closer to such profile which is found in a healthy young adult mammal than to such profile which is found in a mammal having the same chronological age and whose diet was devoid of resveratol. In other words, it has been found in accordance with the present invention that gene expression profiles in adult mammals can be changed towards conformity with expression profiles of such genes found in younger adult mammals by administering to an adult mammal an effective amount of resveratrol, a derivative, metabolite or analogue thereof.

In view of the correlation between the gene expression profile and the physiologic function of an organism mammals treated with resveratrol, a derivative, metabolite or analogue thereof will be in a state of wellness more similar to that of the healthy younger organism than to the average wellness state of an organism of the same age whose diet has not been supplemented with resveratrol. Thus dietary resveratrol, a derivative, metabolite or analogue thereof promote the global wellness state of an organism by inducing physiological changes that will give the organism a younger phenotype. The promotion of wellness by dietary resveratrol in accordance with the invention promotes healthy genes, keeps genes young, improves mental fitness, enhances physical fitness, improves mobility and performance, promotes physical strength and mental strength, provides longevity and healthy aging.

Thus, in one embodiment, the present invention is concerned with a method of promoting the wellness state of a mammal which comprises administering to said mammal an effective amount of resveratrol, a derivative, metabolite or analogue thereof.

In a further embodiment, the present invention is concerned with a method of changing gene expression profiles in adult mammals towards conformity with expression profiles of such genes found in younger adult mammals which comprises administering an adult mammal an effective amount of resveratrol, a derivative, metabolite or analogue thereof.

In yet another embodiment, the present invention is concerned with the use of resveratrol, a derivative, metabolite or analogue thereof, for the manufacture of a nutraceutical composition for promoting the wellness state of a mammal, and for changing gene expression profiles in adult mammals towards conformity with expression profiles of such genes found in younger adult mammals.

The term « nutraceutical » as used herein refers to compositions for use in both the nutritional and pharmaceutical field of application. Thus, a nutraceutical compositions can be a supplement to food and beverages, or a pharmaceutical formulations for enteral or parenteral application which may be solid formulations such as capsules or tablets, or liquid formulations, such as solutions or suspensions. The term « food » is used herein to also include animal feed. As will be evident from the foregoing, the term nutraceutical composition also comprises food and beverages containing the above-specified active ingredients.

More specific embodiments of the present invention include, but are not limited to, the use of resveratrol, a derivative, metabolite or analogue thereof, for
Preventing imbalances in homeostasis by improving the cellular metabolism and performance such improving the body performance;
Maintaining body mobility, Improving physical and mental fitness and strength;
Maintaining the regenerative and repair capacity of an organism by enhancing the ability of that organism to maintain a young state;
Maintaining organ function by avoiding physiological abnormality and/ or biochemical irregularity causing disorders; and
Promoting the ability of an organism to adapt to a changing environment;

The effects of resveratrol, a derivative, metabolite or analogue thereof on gene expression profiles can be determined by methods known per se, e.g., as described in more detail below.

### Animals and dietary manipulations

Male B6C3F₁ mice (6-7 weeks of age) were purchased from Harlan Sprague Dawley. Mice were housed singly and provided water *ad libitum*. The control group (OC, N=5) was fed 98 kcal/week of modified AIN-93M semi-purified diet (Bio-serv, Frenchtown, NJ), which provides approximately 15% fewer calories than the average *ad libitum* dietary intake. The treatment group (RES, N=5) was fed the same caloric intake as controls, but were supplemented with resveratrol 50mg/kg diet (w/w) (3,4',5-Trihydroxy-trans-stilbene; Sigma) from 14 months of age. Animals (OC and RES) were sacrificed at 30 months of age. Young animals were sacrificed at 5 months of age (YC, N=5). Hearts from all abovementioned groups were collected, immediately frozen in liquid nitrogen and stored at -80 °C until analysis.

### Target RNA preparation and high-density oligonucleotide array hybridizations

Total RNA was extracted from frozen tissue by using TRIZOL reagent (Life Technologies, Grand Island, NY). Polyadenylate [poly(A)⁺] RNA was purified from total RNA with oligo-dT-linked oligotex resin (Qiagen, Valencia, CA). One µg of poly(A)⁺ RNA was converted into double-stranded cDNA (ds-cDNA) by using the SuperScript Choice System (Life Technologies), with an oligo-dT primer containing a T7 RNA polymerase promoter (Genset, La Jolla, CA). Ds-cDNA was extracted with phenol-chloroform-isoamyl alcohol and precipitated with pellet paint co-precipitant (Novagen, Madison, WI). Biotin-labeled RNA was synthesized *in vitro* using the BioArray High Yield RNA Transcript Labeling Kit (Enzo, Farmingdale, NY). The biotin-labeled antisense cRNA was then purified using the RNeasy affinity column (Qiagen) and fragmented randomly. The hybridization cocktail (200 µl) containing 10 µg of fragmented cRNA was injected into the mouse Genome 430 2.0 DNA microarray (Affymetrix, Santa Clara, CA). After hybridization, the gene chips were washed and stained in a fluidic station (Model 800101, Affymetrix) with a signal amplification protocol using antibody. DNA chips were scanned at a resolution of 3 µm, twice, using a Hewlett-Packard GeneArray Scanner (Model 900154, Affymetrix) and the averaged images were used for further analysis.

### Data Analysis

Gene expression data were obtained using the Affymetrix Mouse Genome 430 2.0 array containing 45,101 probe sets. All steps, as detailed below, were performed using the most recent version of probe set (Mouse Genome 430. 2.0 array probe set annotations available from Affymetrix at 23 August 2005). Signal intensity was determined using Affymetrix's GeneChip Operating Software version 1.3. Data analysis was performed using Genedata Expressionist^{®} Pro, version 2.0.

### Step 1 Data acquisition and quality control

All data was imported into Genedata Expressionist^{®} Pro. Refiner for quality control, using Diagnose with reference module. Samples that showed a high degree of variability from other similarly treated biological replicates were removed from the data set and excluded from further analysis. Two chips (one in the YC group, one in the RES group) were eliminated due to abnormal data distribution patterns. 18 chips were further analysed using Genedata Expressionist^{®} Pro. Analyst.

Present and marginal signals were selected for further processing using the Affymetrix quality value of 0.065 (threshold for marginal expression). All data was normalized to the median of each chip.
In the further analyses, chips derived from the same treatment groups were grouped together and, for each probe, the group average was set as the median of the group. Only signals where at least 3 out of 5 chips (or 3 out of 4) were present/marginal were used for further selection.

### Step 2 Delete ambiguous probe sets

According to Affymetrix's "Data Analysis Fundamentals" manual (http://www.affymetrix.com/support/downloads/manuals/data_analysis_fundamentals_ma nual.pdf), probe sets that contain the text "_x_at" or "_s_at" do not confidently query a single gene, thus they were filtered from the data set. In addition, probe sets not querying well-characterized genes were eliminated from further analysis. Examples include probe sets representing cDNA sequences, expressed sequence tags (ESTs), RIKEN cDNA sequences, DNA segments or hypothetical proteins.

### Step 3 Statistical analysis

### 1) genes changed with age

Genes which changed with age (ageing markers) were selected by comparing YC and OC groups. To determine if there was a change in the expression of a gene with resveratrol, comparisons were made between OC and RES mice, respectively. Significantly changed genes were selected using the n-fold test, with change > 1.25-fold or consistently present in one group but absent in other groups, in combination with the two-sample test, where significance was tested at P<0.01 (both the t-test and Welch test were used to calculate the statistical significance).

### 2) genes unchanged with age

The genes which were unchanged with age (non-ageing markers) were selected by comparing YC and OC groups, first filtered with a variance <0.05, then further selected with fold of change <1.25-fold, plus 2-group test, where significance was tested at p>0.01 (i.e. significantly unchanged between YC and OC groups). Similarly, significantly-changed genes were determined using either fold of change (>1.25-fold) or consistently present in one group but absent in other groups. Two-tailed t-test and Welch test were both used to calculate the statistical significance, where P<0.01 was considered as a significant change.

### Step 4 Classification using supervised learning

Classification is a complex task which is divided into two phases, supervised learning and a test phase. The goal of classification is to predict an output variable (in this case, age) given an individual's input data. The supervised learning phase involves the application of an algorithm to training data, with the goal of learning rules by which the output variable can be predicted, while the test phase constitutes predictions being made for novel individuals, applying the rules collected in the learning phase. It is also possible to make predictions based on supplied training data.

For the classification analysis, supervised learning using Support Vector Machine (SVM) was performed based on supplied training data, "Young (YC)" and "Old (OC)" groups, to find rules for predicting the output variable "Age". A cleaned probe set (see above) was employed. Results from the resveratrol group were then analysed using the Classification function for prediction of the output variable, "Age". All 4 chips were classified as Young. A numeric value of classification output ranging from +1 to -1 was assigned to each chip, where +1 indicates a perfect match for a specific category, and -1 indicates a mismatch to the highest extent.

**Table I: This table contains the numerical values of the classifier outputs from each chip; average (mean) and standard deviation are calculated for each group. The resveratrol group had a mean classification output of 0.34855, which is clearly much closer to the value calculated for the young group, than that of the old group.**

| **Group** | **Experiment** | **Age (Young=1, Od=-1)** | **Average of** | **g STEV** |
|---|---|---|---|---|
| YC | DSh-yc2 | 1 | 0.9998575 | 0.000202711 |
| | DSh-yc3 | 1 | | |
| | DSh-yc4 | 0.99986 | | |
| | DSh-yc5 | 0.99957 | | |
| OC | DSh-oc1 | -1 | -1.023992 | 0.053670106 |
| | DSh-oc2 | -0.99996 | | |
| | DSh-oc3 | -1 | | |
| | DSh-oc4 | -1.12 | | |
| | DSh-oc5 | -1 | | |
| RES | DSh-Re1 | 0.51896 | 0.34855 | 0.140348421 |
| | DSh-Re2 | 0.25584 | | |
| | DSh-Re5 | 0.21368 | | |
| | DSh-Re7 | 0.40572 | | |

The genome-wide gene expression in heart tissue in groups of young, old and resveratrol-fed mice was monitored using the mouse Affymetrix gene array. Among the 26,000 probe sets available on the chip, 1285 genes were significantly changed between the groups of young mice and old mice (at least 1.25-fold, where p<0.01). Among them, 501 genes were up- and 724 were down-regulated in the group of old mice. Within the 1285 genes, the expression of 588 gene in the resveratrol treated group were changed in the direction of the group of younger adult mice (45.7%, see table III), indicating a significant effect of resveratrol in maitaining the heart in a healthy and young state To summarize the scope of the resveratrol effect, in the 1023 above mentioned genes changed by resveratrol treatement, 342 resembled or exceeded the expression level of the young group (58.1 %); 62 genes were at a level of 80- 90% compared to the young group and 53 genes reached a level of 70- 80%. Therefore, in spite of being of the same biological age as the mice in the group of old animals, old animals fed a resveratrol-supplemented diet showed a gene profile surprisingly close to that of the young animals.

**Table II : Gene expression levels in resveratrol-fed old mice**

| **Effect*** | **number of genes changed by resveratrol** |
|---|---|
| >100% | 342 |
| 90- | |
| 100% | 62 |
| 80-90% | 53 |
| 70-80% | 48 |
| 60-70% | 39 |
| <60% | 44 |
| Sum | 588 |

| | |
|---|---|
| Effect* : Gene expression levels in resveratrol-fed old mice expressed as percentage of gene expression levels observed in untreated young mice. | |

It is well documented in the literature that the ageing process is accompanied by a decreased function of protein synthesis and protein folding, which leads to an imbalance of protein turnover, especially in muscle. Exercise has been shown to increase muscle protein synthesis and mitochondrial function in the elderly. In addition, protein folding and protein modification is also affected by ageing, as a consequence of physiological and pathological changes. Many diseases, such as Alzheimer's and Parkinson's disease, are associated with abnormal protein modification. In the old animal's group, significant reductions in many genes related to protein synthesis and protein folding (see table I) were observed. In contrast, in old animals fed a resveratrol-supplemented diet, many of these genes were upregulated to or close to the level found in young animals, e.g. genes involved in protein synthesis such as eukaryotic translation initiation factors 4A1, 2, 3, 4E, 4g1. Similarly, genes involved in protein modification, such as f-box only protein 9, homocysteine-inducible, endoplasmic reticulum stress-inducible, ubiquitin-like domain member 1 and ubiquitin specific protease 3 were all significantly down-regulated in the group of old animals, while these effects were reversed in the resveratrol group.(see Table III).

In conclusion, the present study showed that the gene expression profile of an adult mammal treated orally with resveratrol is closer to a healthy younger adult mammal than to a mammal having the same chronological age. This younger gene expression profile in resveratrol treated animals promotes the state of welleness in the animals.

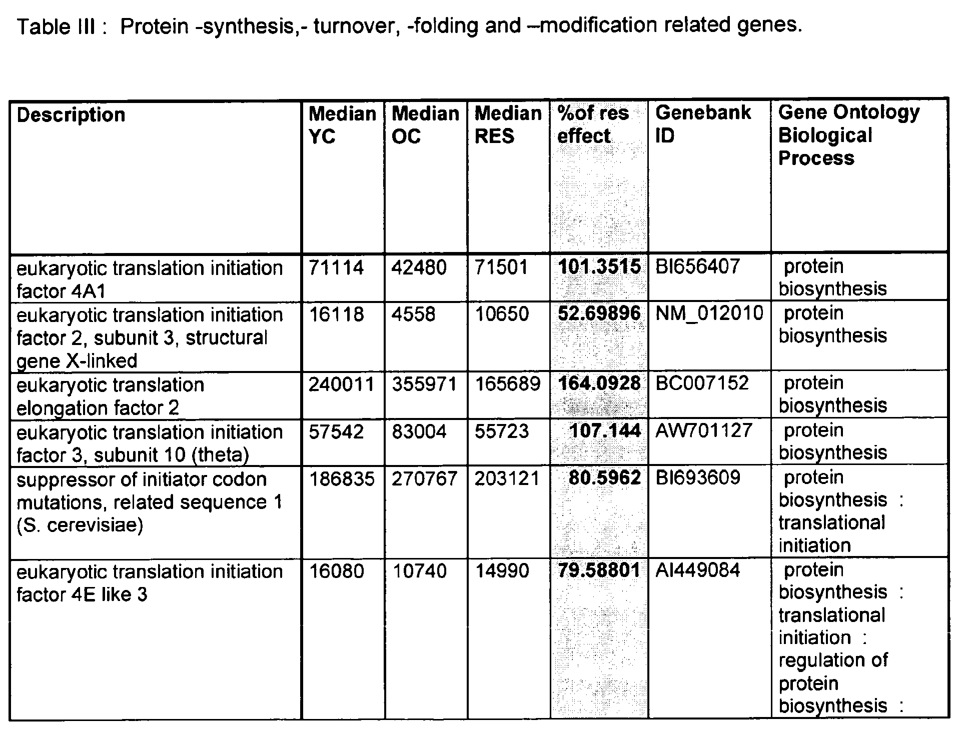

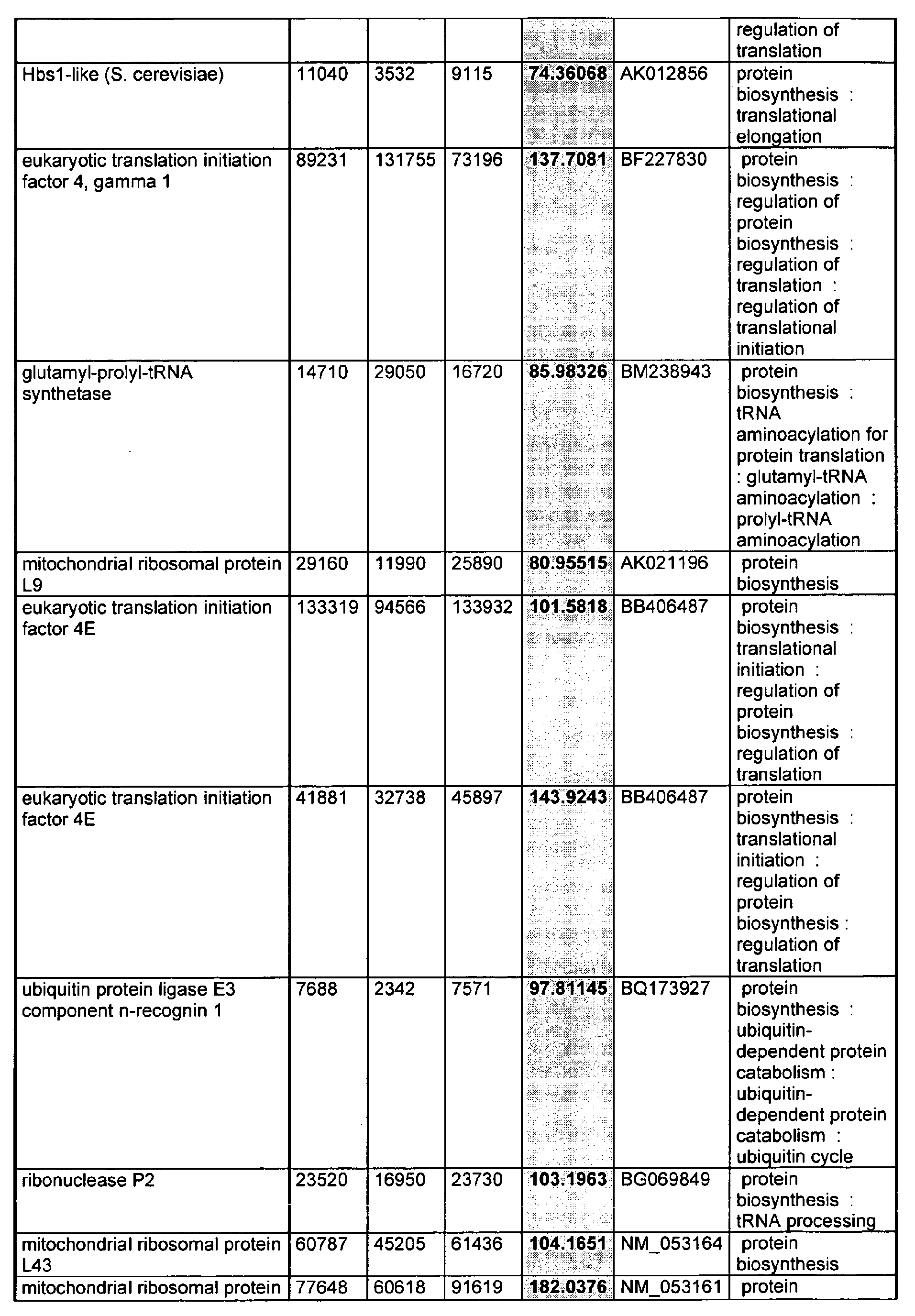

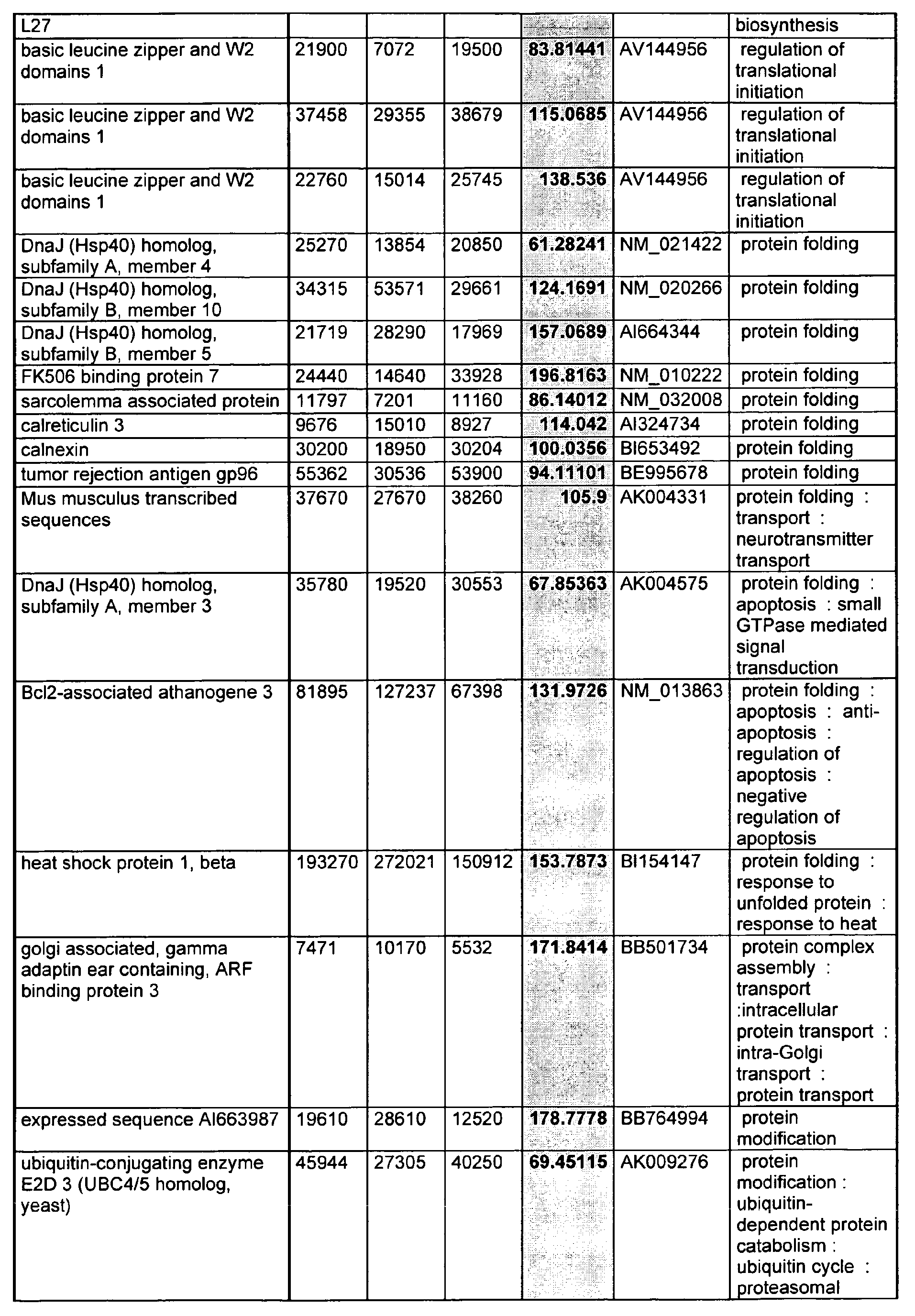

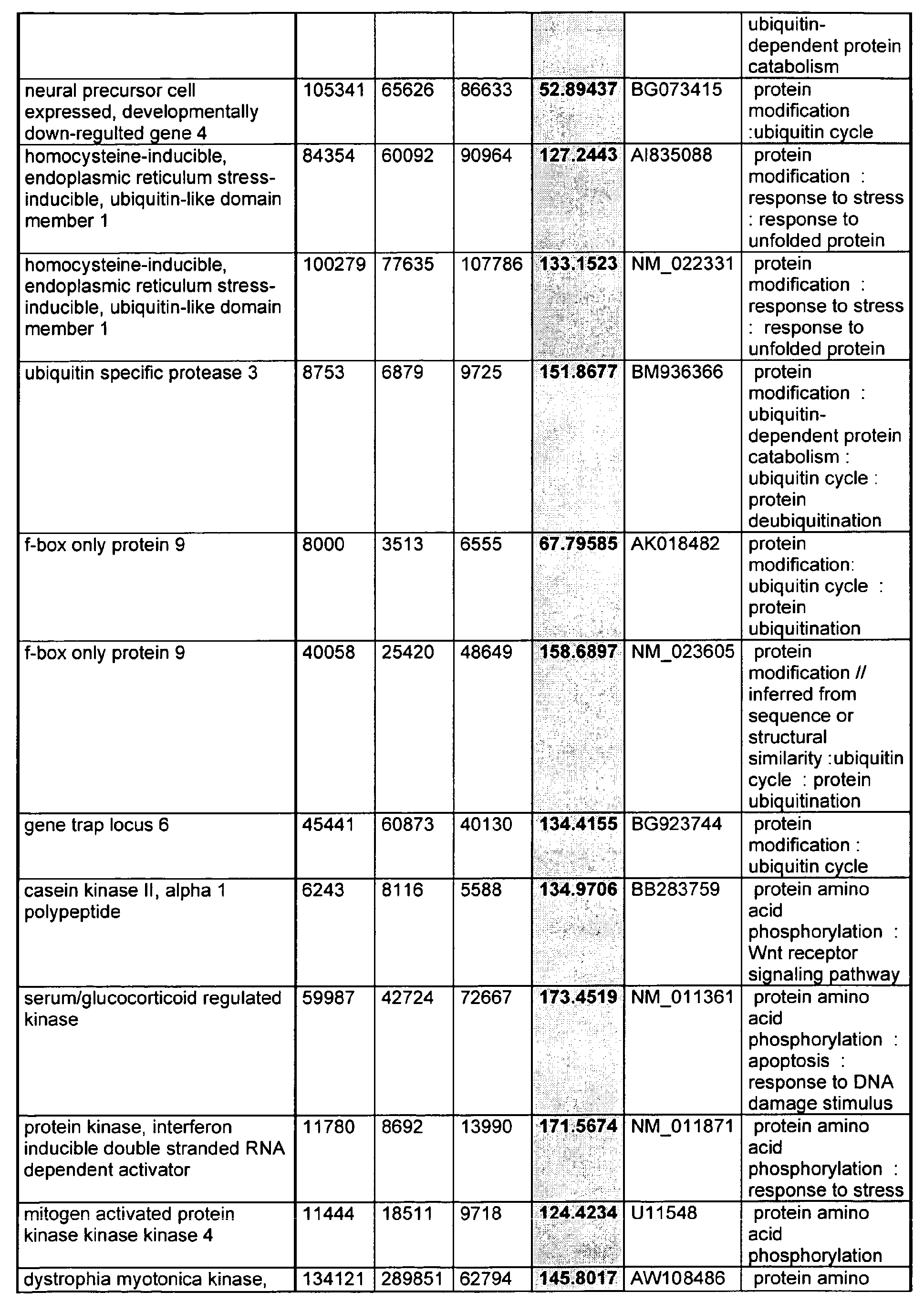

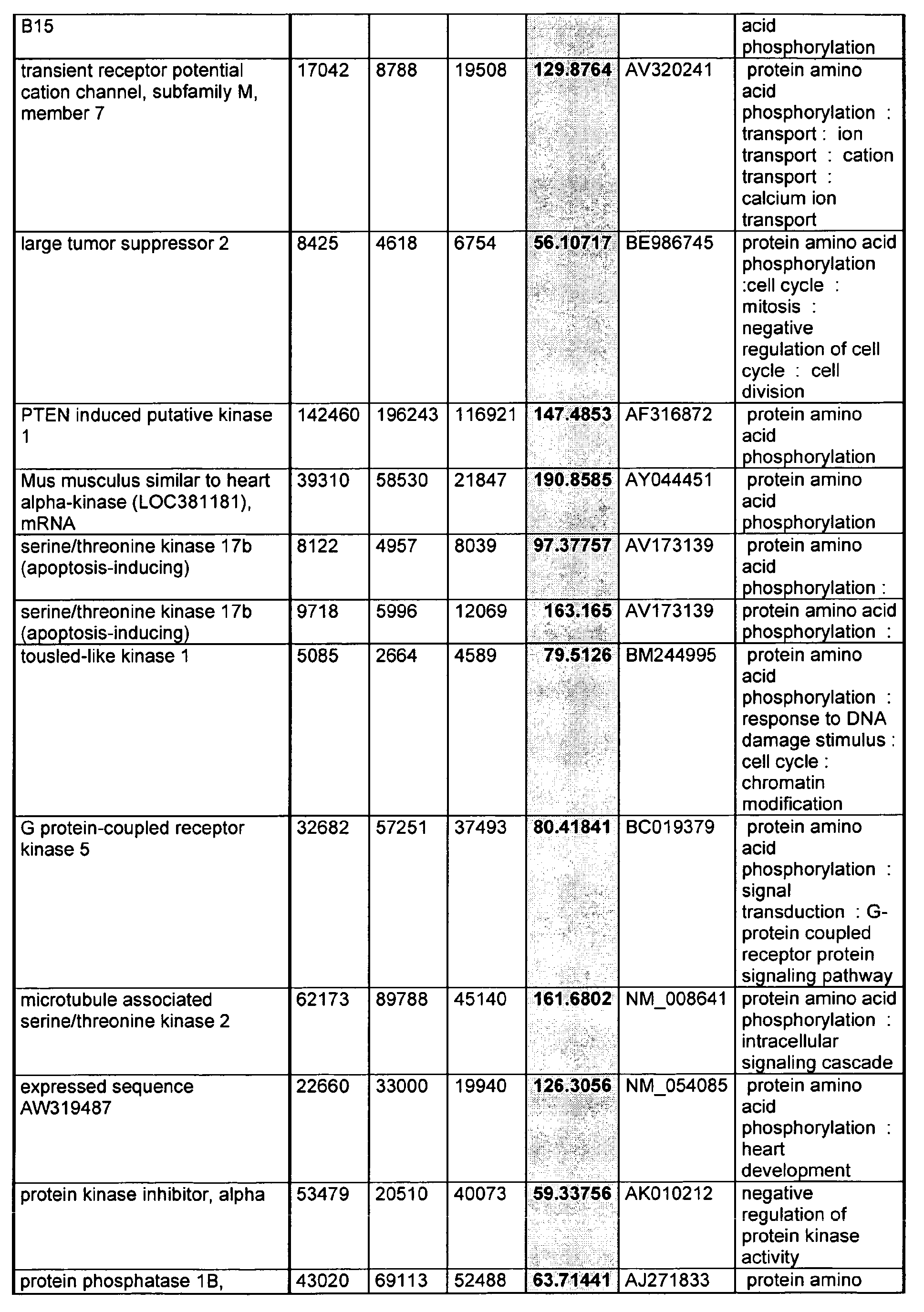

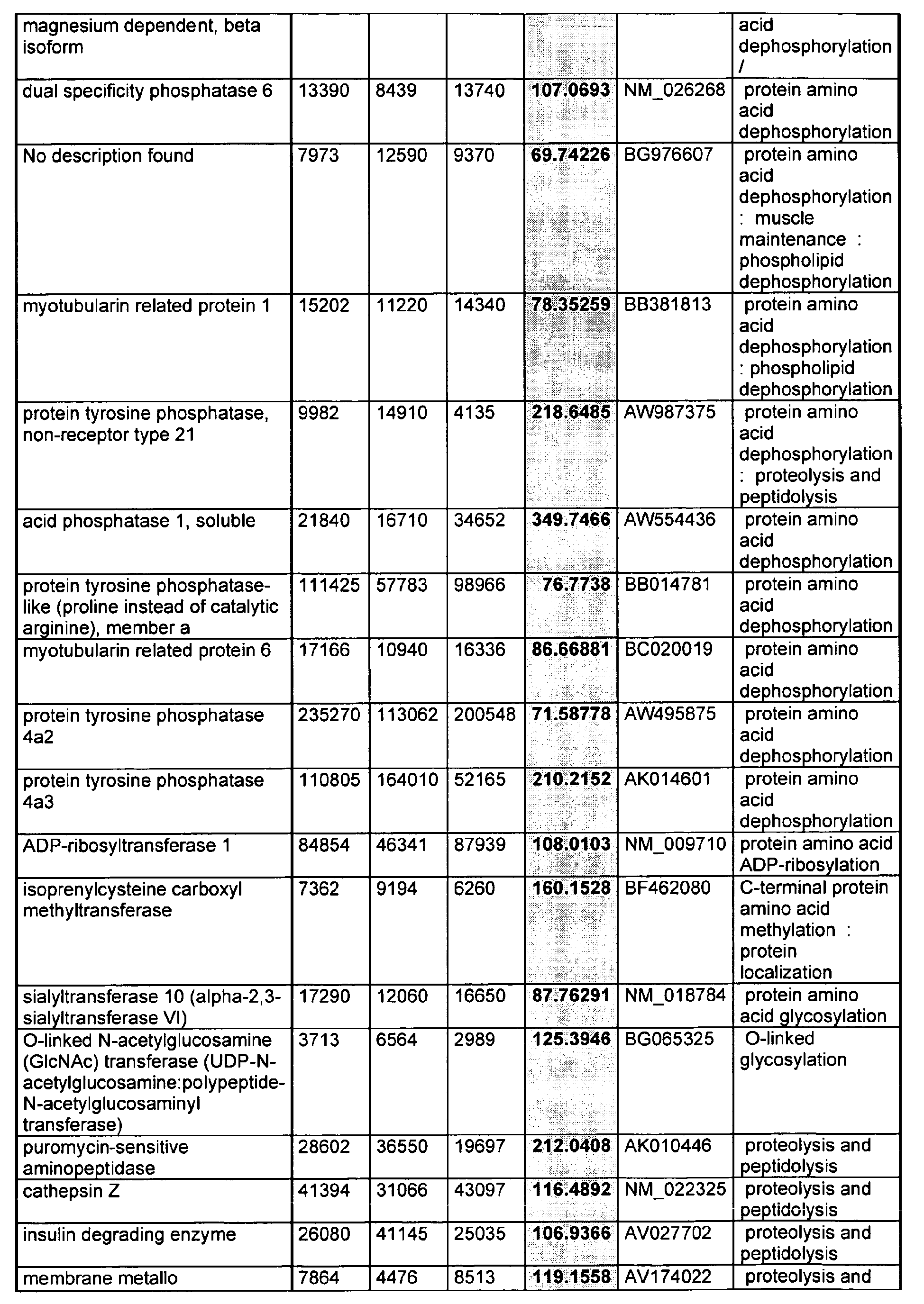

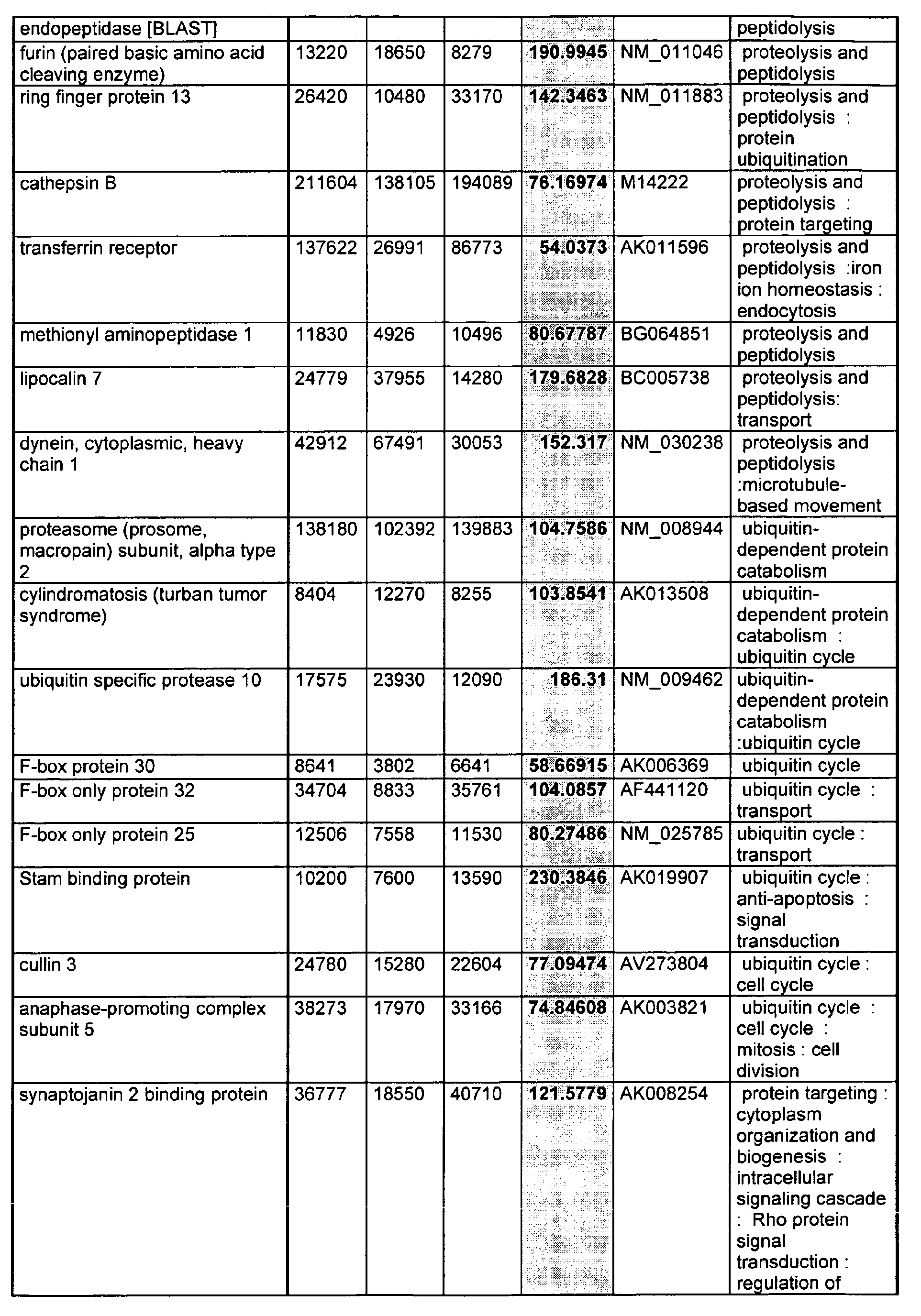

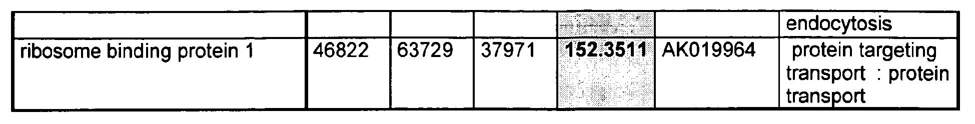

For the purposes of the invention, the dosage requirements for resveratrol, a derivative, metabolite or analogue are not narrowly critical. Amounts of up to about 30 mg/kg body weight per day or even higher, depending of the nature of the mammal concerned and its condition and requirements may be administered. Thus, for a human adult (weighing about 70 kg) the dosage may be up to about 2000 mg/day. In a particular embodiment of the invention, the dosage for a human adult (weighing about 70 kg) is up to about 500 mg/day, especially up to about 500 mg/day. Suitably, the dosage is no less than 0.5 mg. In a particular embodiment of the invention, the dosage for a human adult (weighing about 70 kg) is no less than 2 mg., especially is no less than 5 mg. If administered in a food or beverage the amount of resveratrol, a derivative, metabolite or analogue thereof contained therein is suitably no less than about 0.2 mg per serving. In another embodiment of the invention such amount is no less than 2 mg per serving. On the other side, resveratrol, a derivative, metabolite or analogue thereof may be administered in a food or beverage in an amount of up to 100, 200 or 500 mg per serving. If resveratrol, a derivative, metabolite or analogue thereof is adminstered as a pharmaceutical formulation such formulation may contain up to about 100, 200 or 500 mg per solid dosage unit, e.g., per capsule or tablet, or up to about 2000 mg per daily dose of a liquid formulation. If resveratrol is used as an extract from natural sources the above dosage figures refer to the amount of pure resveratrol contained in the extract.

The term "serving" as used herein denotes an amount of food or beverage normally ingested by a human adult with a meal at a time and may range, e.g., from about 100 g to about 500 g.

For the purposes of the present invention resveratrol, a derivative, metabolite or analogue thereof may be administered as a nutritional supplement, e.g., as an additive to a multivitamin preparations comprising vitamins and minerals which are essential for the maintenance of normal metabolic function Resveratrol, a derivative, metabolite or analogue thereof may be adminstered also as a pharmaceutical composition, preferably for enteral application, which may be solid or liquid galenical formulation. Examples of solid galenical formulations are tablets, capsules (e.g. hard or soft shell gelatin capsules), pills, sachets, powders, granules and the like which contain the active ingredient together with conventional galenical carriers. Any conventional carrier material can be utilized. The carrier material can be organic or inorganic inert carrier material suitable for oral administration. Suitable carriers include water, gelatin, gum arabic, lactose, starch, magnesium stearate, talc, vegetable oils, and the like. Additionally, additives such as flavouring agents, preservatives, stabilizers, emulsifying agents, buffers and the like may be added in accordance with accepted practices of pharmaceutical compounding. While the individual active ingredients are suitably administered in a single composition they may also be administered in individual dosage units.

### The following Examples illustrate the invention further

Pharmaceutical compositions may be prepared by conventional formulation procedures

### Example 1

### Soft gelatin capsule

Soft gelatin capsules are prepared by conventional procedures containing as active ingredient 30 mg of resveratrol per capsule.

### Example 2

### Hard gelatin capsule

Hard gelatin capsules are prepared by conventional procedures containing as active ingredient 20 mg of resveratrol per capsule.

### Example 3

### Tablet

Tablets are prepared by conventional procedures containing as active ingredient 10 mg of resveratrol per tablet, and as excipients microcrystalline cellulose, silicone dioxide (SiO2), magnesium stearate, crospovidone NF (which is a disintegration agent) ad 200 mg.

### Example 4

Food items may be prepared by conventional procedures containing resveratrol in an amount of 0.2 mg to 200 mg per serving. Examples of such food items are soft drinks, bread, cookies, yogurth, ice cream, and sweets.

For example an orange-Lemon juice drink, containing 10% juice and resveratrol is prepared from the follwoing ingredients:

| Ingredients | [g] |
|---|---|
| Sugar syrup 64°Brix | 156.2 |
| Sodium benzoate | 0.2 |
| Ascorbic acid, fine powder | 0.2 |
| Citric acid 50% w/w | 5.0 |
| Pectin solution 2% w/w | 10.0 |
| Resveratrol | 0.02 |
| Juice compound* | 30.0 |
| Water to | 250.0 |

### Preparation

- Dissolve sodium benzoate in water whilst stirring
- Continue stirring and add sugar syrup, ascorbic acid, citric acid, pectin solution, juice compound, one after the other. Do not use a high speed mixer
- Dilute the bottling syrup with (carbonated) water to one liter of beverage

| *Ingredients Juice compound | [g] |
|---|---|
| Orange juice concentrate 65°Brix | 483.3 |
| Lemon Juice Concentrate 45°Brix | 173.3 |
| Oily orange flavour | 5.0 |
| β-Carotene 10% CWS as 10% stocksolution | 10.0 |
| Deionized water | 328.4 |

### Preparation of juice compound

- Add the deionized water to the juice concentrates, stir gently and allow the juice concentrates to hydrate.
- Add the oily flavour and β-Carotene 10% CWS stocksolution and pre-emulsify in a rotor-stator-homogenizer.
- Homogenize in a high-pressure homogenizer at 200 bar.

### Addition of β-Carotene 10% CWS

β-Carotene 10% CWS should be added to the juice compound as a 1 - 10 % stocksolution in deionized water

### Example 5

A reconvalescent 70 year old person weighing 55 kg is administered resveratrol at a dosage regimen of 20 mg per day for a duration of 2 months.

### Example 6

A person aged 55 years weighing 70 kg intending to participate in a sporting event is administered 30 mg of resveratrol per day for 3 months before said event.

### Example 7

A person aged 60 years weighing 75 kg complaining of frequent episodes of tiredness and general lack of motivation is administered 50 mg of resveratrol per day for 3 months.

## Claims

1. The use of resveratrol, a derivative, metabolite or analogue thereof for the manufacture of a nutraceutical composition for promoting the wellness state of a mammal.

2. The use of resveratrol, a derivative, metabolite or analogue thereof for the manufacture of a nutraceutical composition for changing gene expression profiles in adult mammals towards conformity with expression profiles of such genes found in younger adult mammals

3. The use as in claim 2 wherein the genes are those involved in the maintenance and/or improvement of well-being of mammals.

4. The use as in claim 2 or 3 wherein the genes are those involved in the maintenance of physical and mental health of a mammal.

5. The use as in any one of claims 2 to 4 wherein the genes are those involved in the maintenance of normal or improvement of abnormal physiological values of at least one of blood cholesterol (lipids), blood triglyceride lipids, blood LDL and HDL and the LDL to HDL ratio, blood glucose, liver function, heart rate, protein, vitamin and mineral metabolism, immune system natural killer cell (NK) activity, immune system vitality and proportion of NK cells, immune cells (NK, B and T-cell counts) and/or immune T-cell helper/suppressor ratio.

6. The use as in any one of claims 2 to 5 wherein the genes are those involved in apoptosis, cell adhesion, cell growth and maintenance, cytoskeletal organization, embryonal development, electron transport, endo-exophagocytosis, inflammation/immune response, metabolism of carbohydrates, fatty acids, lipids, nucleic acids, TCA cycle, protein folding, protein synthesis, protein ubiquination, proteolysis, response to stress, signal transduction, transcription, or transport.

7. The use as in any one of claims 2 to 6 wherein the genes are those involved in expression of IgF1r, Bcl2 antagonist, cyclooxygenase, especially genes involved in protein synthesis, turnover and modification, such as eIF4A, 4E, 4gamma1,eIF3subunit10, eukaryotic translation elongation factor 2, mitochondrial ribosomal protein L43, L27, ARF binding protein3, f-box only protein 9, DnaJ (Hsp40 homolog, and/or Hsp1 beta.

8. The use as in any one of claims 1 - 7 of resveratrol of synthetic origin.

9. The use as in any one of claims 1 - 7 of resveratrol as a resveratrol-containing extract from natural resveratrol sources.

10. The use as in claims 1 - 9 wherein the nutraceutical composition is a food or beverage.

11. The use as in claim 10 wherein the food or beverage contains resveratrol, a derivative, metabolite or analogue thereof in an amount sufficient to provide no less than 0.2 mg per serving.

12. The use as in claim 10 wherein the food or beverage contains resveratrol, a derivative, metabolite or analogue thereof in an amount sufficient to provide no less than 2 mg per serving.

13. The use as in claim 1 - 9 wherein the nutraceutical composition is a dosage unit composition.

14. The use as in claim 13 wherein the dosage unit contains no less than 0.5 mg of resveratrol, a derivative, metabolite or analogue thereof.

15. The use as in claim 13 wherein the dosage unit contains no less than 5 mg of resveratrol, a derivative, metabolite or analogue thereof.

16. A method of maintaining and/or promoting the wellness state of a mammal which comprises administering to said mammal an effective amount of resveratrol, a derivative, metabolite or analogue thereof.

17. A method of changing gene expression profiles in adult mammals towards conformity with expression profiles of such genes found in younger adult mammals which comprises administering an adult mammal an effective amount of resveratrol, a derivative, metabolite or analogue thereof.

## Amended claims

### Amended claims in accordance with Rule 86(2) EPC.

**1.** The use of resveratrol, a derivative, metabolite or analogue thereof for the manufacture of a nutraceutical composition for promoting the wellness state of a mammal.

**2.** The use as in claim 1, wherein resveratrol is of synthetic origin.

**3.** The use as in claim 1, wherein resveratrol is a resveratrol-containing extract from natural resveratrol sources.

**4.** The use as in any one of claims 1 - 3, wherein the nutraceutical composition is a food or beverage.

**5.** The use as in claim 4, wherein the food or beverage contains resveratrol, a derivative, metabolite or analogue thereof in an amount sufficient to provide no less than 0.2 mg per serving.

**6.** The use as in claim 4, wherein the food or beverage contains resveratrol, a derivative, metabolite or analogue thereof in an amount sufficient to provide no less than 2 mg per serving.

**7.** The use as in any one of claims 1 - 3, wherein the nutraceutical composition is a dosage unit composition.

**8.** The use as in claim 7, wherein the dosage unit contains no less than 0.5 mg of resveratrol, a derivative, metabolite or analogue thereof.

**9.** The use as in claim 7, wherein the dosage unit contains no less than 5 mg of resveratrol, a derivative, metabolite or analogue thereof.

**10.** A method of promoting the wellness state of a mammal which comprises the manufacture of a nutraceutical composition as defined in any one of claims 1 - 9 meant for administration of an effective amount of resveratrol, a derivative, metabolite or analogue thereof to said mammal.
